Europäisches Patentamt

European Patent Office

Office européen des brevets

(19)

(11) Veröffentlichungsnummer : **0 091 514**
B1

(12)  # EUROPÄISCHE PATENTSCHRIFT

(45) Veröffentlichungstag der Patentschrift :
16.06.87

(21) Anmeldenummer : 82110943.6

(22) Anmeldetag : 26.11.82

(51) Int. Cl.⁴ : **G 02 F   1/13, A 61 F   9/06**

(54) **Lichtschutzfilter, insbesondere für Schweissschutzschilder oder Schweissschutzbrillen.**

(30) Priorität : 14.04.82 CH 2264/82

(43) Veröffentlichungstag der Anmeldung :
19.10.83 Patentblatt 83/42

(45) Bekanntmachung des Hinweises auf die Patenterteilung : 16.06.87 Patentblatt 87/25

(84) Benannte Vertragsstaaten :
**AT BE CH DE FR GB IT LI LU NL SE**

(56) Entgegenhaltungen :
EP-A- 0 005 417
DE-A- 2 442 998
FR-A- 2 178 052
FR-A- 2 293 188
GB-A- 1 589 972
US-A- 4 039 254
US-A- 4 279 474

(73) Patentinhaber : **Eggenschwiler, André M.**
**Obere Bühlstrasse 15**
**CH-8700 Küsnacht (CH)**

(72) Erfinder : **Bruhin, Rolf**
**Usterstrasse 106c**
**CH-8621 Wetzikon 4 (CH)**
Erfinder : **Eggenschwiler André M.**
**Obere Bühlstrasse 15**
**CH-8700 Küsnacht (CH)**

(74) Vertreter : **Rottmann, Maximilian R.**
**c/o Rottmann + Quehl AG Glattalstrasse 37**
**CH-8052 Zürich (CH)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 091 514 B1

### Beschreibung

Die vorliegende Erfindung bezieht sich auf ein Lichtschutzfilter nach dem Oberbegriff des Patentanspruches 1 bzw. nach dem Oberbegriff des Patentanspruches 2.

Aus der DE-A-2 442 998 ist ein Lichtschutzfilter gemäss dem Oberbegriff des Patentanspruches 1 bekannt. Das Sensorelement dieser Anordnung liegt dabei hinter dem transmissionsgesteuerten Filterelement ; es ist aber aus dieser Druckschrift nirgends ersichtlich, einerseits of ein UV- bzw. IR-Sperrfilter vorgeschaltet ist, andererseits ob das Sensorelement gegen Umgebungs- bzw. Streulicht abgeschirmt ist. Die Praxis zeigt, dass ein derartiges Lichtschutzfilter, wie in der genannten DE-A-2 442 998 beschrieben, sehr empfindlich gegen Störeinflüsse ist. Diese Störeinflüsse sind zum einen die UV-Strahlung durch Sonnenlicht, die IR-Strahlung durch benachbarte heisse Körper oder die Arbeit eines Schweissers, der daneben seiner Beschäftigung nachgeht. All diese Störfaktoren bewirken, dass das Lichtschutzfilter unbeabsichtigt dunkelgesteuert wird.

Die vorliegende Erfindung hat zur Aufgabe, ein gattungsgemässes Lichtschutzfilter so zu verbessern, dass eine verbesserte Betriebssicherheit sowie eine verbesserte Anpassung an unterschiedliche Beleuchtungsverhältnisse erreicht wird. Diese Aufgabe wird durch die Merkmale im Kennzeichen des Patentanspruchs 1, sowie bei einem Lichtschutzfilter der im Oberbegriff des Patentanspruchs 2 genannten Art durch die Merkmale im Kennzeichen dieses Anspruchs gelöst. Die letzteren Merkmale gewährleisten, dass eine vergleichsweise grosse Restlichtmenge auf den Sensor auftrifft, was der Zuverlässigkeit der Transmissionsregelung zugute kommt, währenddem gleichzeitig für den Benutzer, durch die zwei hintereinandergeschalteten Filterelemente, eine starke Abdunkelung geboten wird.

Die Massnahme nach Patentanspruch 3, wonach der Sensor für die Transmissionssteuerung durch das optoelektrische Wandlerelement gebildet ist, lässt sich sowohl auf die Konstruktion gemäss Patentanspruch 1 als auch auf diejenige gemäss Patentanspruch 2 anwenden und bietet eine unter Umständen sehr erwünschte, konstruktive Vereinfachung.

Im folgenden wird der Erfindungsgegenstand näher erläutert. In den beiliegenden Zeichnungen zeigen die Fig. 1 bis 3 prinzipielle Anordnungen solcher Lichtschutzfilter und die Fig. 4 und 5 je ein Ausführungsbeispiel der Erfindung.

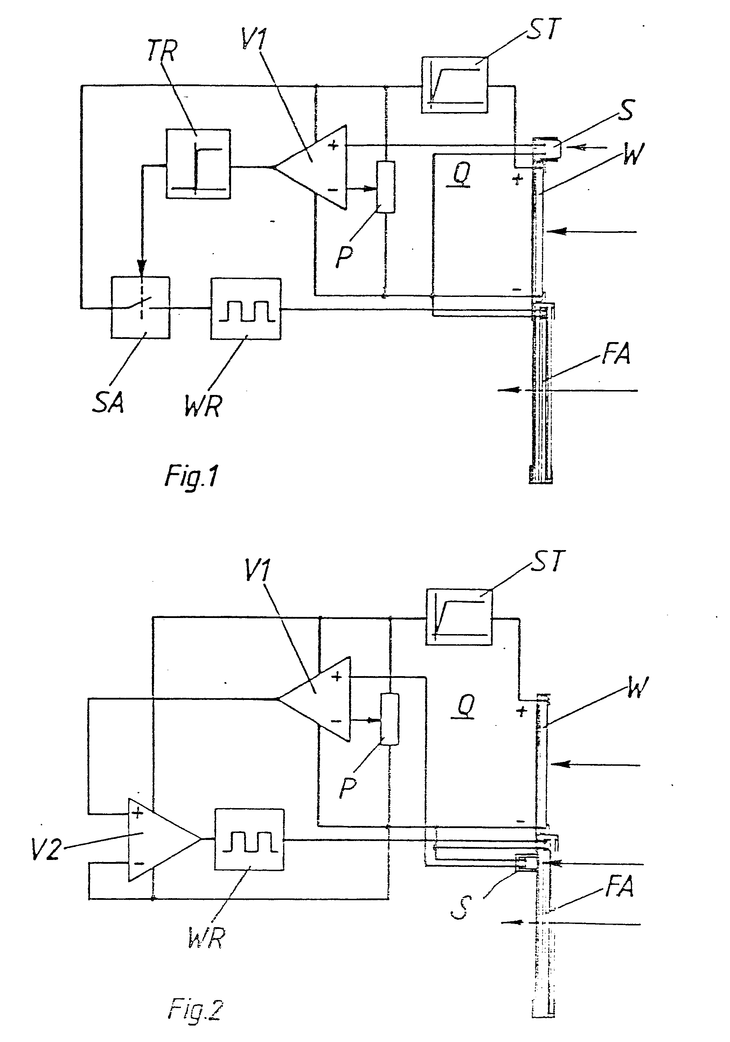

Die Fig. 1 zeigt eine transmissionsgesteuerte Filteranordnung FA an sich bekannter Art, die an eine Speisestromquelle Q für die Transmissionssteuerung angeschlossen ist. Letztere besteht aus einem optoelektrischen Wandler W, der dem Aussenlicht gemäss Pfeil ausgesetzt ist, sowie einem nachgeordneten Stabilisator ST und einem Wechselrichter WR. Eine Sensor- und Steuereinrichtung für die Transmissionsbeeinflussung der Filteranordnung umfasst eine Photozelle als Sensor S, der über einen als Schaltregler mit Sollwerteinstellung durch Potentiometer P wirkenden Differentialverstärker $V_1$ und über einen nachgeordneten Trigger TR einen Analogschalter SA im Speisestromkreis betätigt. Ein solches Lichtschutzfilter ist fremdstromunabhängig, betriebssicher und hinsichtlich seiner Schaltschwelle genau einstellbar.

Bei der Ausführung nach Fig. 2 ist der Sensor S in Lichteinfallsrichtung gemäss Pfeil hinter der transmissionsgesteuerten Filteranordnung FA angeordnet. Dadurch bildet die Sensor- und Steuereinrichtung einen Transmissionsregelkreis mit der Filteranordnung FA als Stellglied. Der Differentialverstärker V1 wirkt als Sollwert-Istwertvergleicher mit Potentiometer P als Sollwertgeber. Ein nachgeordneter Leistungsverstärker V2 speist den Wechselrichter WR. Im übrigen stimmt die Speisestromquelle Q mit der Ausführung nach Fig. 1 überein.

Fig. 2 zeigt das Prinzip einer stetiger Transmissionsregelung, die das durchtretende Licht in seiner Intensität in gewissen Grenzen annähernd konstant halten kann. Die Einrichtung ermöglicht also z. B. bei der Anwendung für Schweisszwecke oder dergl. ein Arbeiten mit annähernd gleichbleibender Beleuchtungsintensität für das Auge des Benutzers, während die Objektbeleuchtung stark schwanken kann.

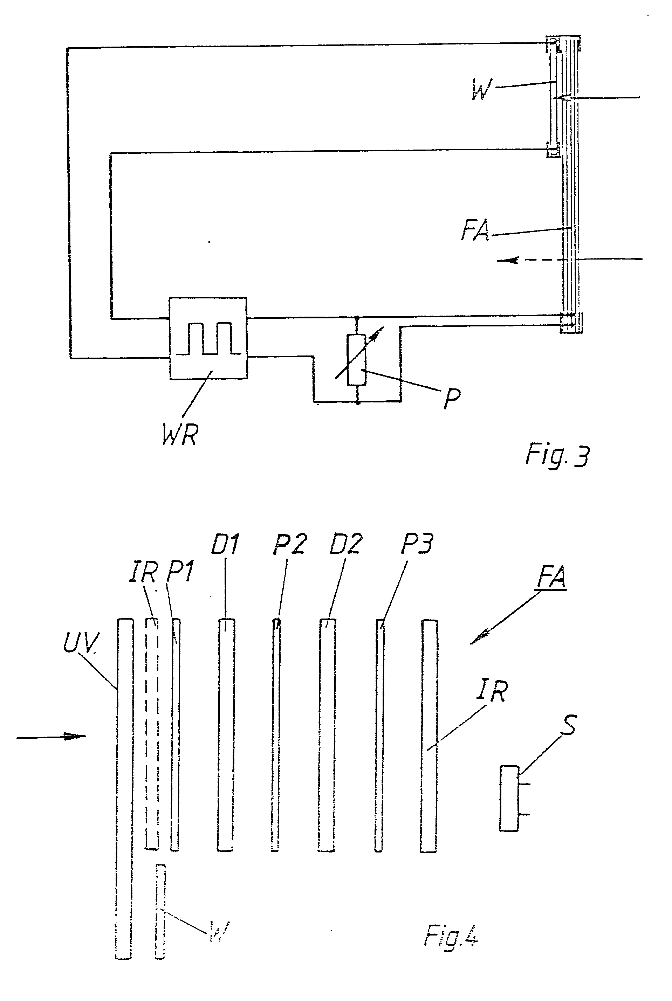

Die Ausführung nach Fig. 3 ermöglicht ebenfalls eine Transmissionsregelung mit annähernd konstanter Beobachtungslichtintensität, jedoch mit vereinfachtem Bau- und Schaltungsaufwand. Hierzu ist der optoelektrische Speisestromwandler W gleichzeitig als Sensor für die Transmissionsregelung eingesetzt und hinter der Filteranordnung FA im durchfallenden Licht angeordnet. Der Wechselrichter WR ist dem Wandler unmittelbar nachgeordnet. Da der Wandler bei abgedunkeltem Filter mit geringer Lichtintensität arbeitet und demgemäss einen hohen Innenwiderstand aufweist, ist für die Reglereinstellung ein Nebenschlusspotentiometer P in Verbindung mit dem hochohmigen Eingang der Filteranordnung vorgesehen. Diese Ausführung zeichnet sich bei für viele Zwecke ausreichendem Regelverhalten durch niedrigen Aufwand aus.

Fig. 4 zeigt den Aufbau einer Filteranordnung FA mit parallelgeschaltetem Wandler W und nachgeordnetem Sensor S in schematisch auseinandergezogener Darstellung. Der Wandler W liegt nur hinter einem UV-Filter UV, so dass er gemäss seinem Empfindlichkeitsspektrum energetisch optimal betrieben werden kann, während ein Infrarotfilter IR für den zusätzlichen Strahlenschutz des Benutzers der Filteranordnung nachgeschaltet ist. Mit Vorteil kann das Filter IR auch — wie strichliert angedeutet — der Filteranordnung FA vorgeschaltet werden. Im übrigen umfasst die Filteranordnung in an sich üblicher Weise zwei hintereinandergeschaltete Drehzellen D1 und D2 sowie zugehörige Polarisatoren P1 bis P3.

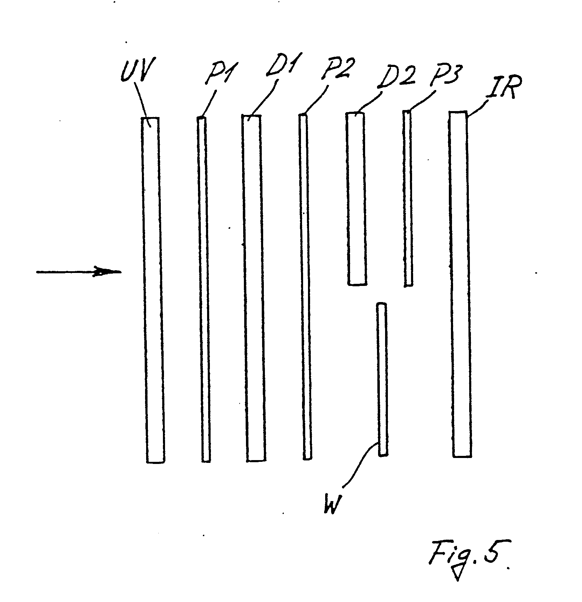

Bei der Ausführung nach Fig. 5, die gemäss Fig. 4 zwei hintereinandergeschaltete Drehzellen D1 und D2 aufweist, ist der als Sensor für die Transmissionsregelung wirkende Speisestromwandler W nur hinter einer der beiden Drehzellen angeordnet, erhält also relativ grössere Restlichtintensität im abgedunkelten Aussteuerzustand und arbeitet somit zuverlässiger.

**Patentansprüche**

1. Lichtschutzfilter für Schweißschutzschilder oder Schweißschutzbrillen, mit einem bezüglich seiner optischen Transmission elektrisch steuerbaren Filterelement, das mit einer Transmissionssteuereinrichtung in Wirkverbindung steht, welche mindestens einen in Lichteinfallsrichtung gesehen hinter dem Filterelement angeordneten, lichtempfindlichen Sensor umfasst und welche das Filterelement in gegensinniger Abhängigkeit von der Intensität des auf den Sensor fallenden Lichtes auf unterschiedliche Transmissionswerte einstellt, wobei als Speisestromquelle für die Transmissionssteuerung ein optoelektrischer Wandler vorgesehen ist, dadurch gekennzeichnet, dass der Sensor hinter einem IR-Sperrfilter und gegebenenfalls hinter einem UV-Sperrfilter angeordnet ist.

2. Lichtschutzfilter für Schweißschutzschilder oder Schweißschutzbrillen, mit einer bezüglich ihrer optischen Transmission elektrisch steuerbaren, aus zwei hintereinandergeschalteten Filterelementen bestehenden Filteranordnung, die mit einer Transmissionssteuereinrichtung in Wirkverbindung steht, welche mindestens einen in Lichteinfallsrichtung gesehen hinter der Filteranordnung angeordneten, lichtempfindlichen Sensor umfasst und welche die Filteranordnung in gegensinniger Abhängigkeit von der Intensität des auf den Sensor fallenden Lichtes auf unterschiedliche Transmissionswerte einstellt, wobei als Speisestromquelle für die Transmissionssteuerung ein optoelektrischer Wandler vorgesehen ist, dadurch gekennzeichnet, dass der Sensor hinter einem IR-Sperrfilter und hinter dem ersten, aber vor dem zweiten Filterelement der Filteranordnung sowie gegebenenfalls hinter einem UV-Sperrfilter angeordnet ist.

3. Lichtschutzfilter nach Anspruch 1 oder 2, dadurch gekennzeichnet, dass der Sensor für die Transmissionssteuerung durch das optoelektrische Wandlerelement gebildet ist.

**Claims**

1. Protective light filter for welder's protective shields or welder's protective eye glasses, with a filter element electrically controllable with regard to its optical transmission rate and operationally connected to a transmission rate control circuitry which includes at least one light sensitive sensor arranged behind the filter element as seen in the direction of incident light and setting the filter element to different transmission rate values in reverse dependence of the intensity of the light falling on the sensor, whereby an optoelectric transducer is provided serving as power supply for the transmission rate control circuitry, characterized in that the sensor is mounted behind an infrared rejection filter and, if appropriate, behind an ultraviolet rejection filter.

2. Protective light filter for welder's protective shields or welder's protective eye glasses, with a filter array comprising two filter elements one behind the other electrically controllable with regard to its optical transmission rate and operationally connected to a transmission rate control circuitry which includes at least one light sensitive sensor arranged behind the filter array as seen in the direction of incident light and setting the filter array to different transmission rate values in reverse dependence of the intensity of the light falling on the sensor, whereby an optoelectric transducer is provided serving as power supply for the transmission rate control circuitry, characterized in that the sensor is mounted behind an infrared rejection filter and behind the first but in front of the second filter element and, if appropriate, behind an ultraviolet rejection filter.

3. Protective light filter according to claim 1 or 2, characterized in that the sensor of the transmission rate control circuitry is constituted by the optoelectric transducer element.

**Revendications**

1. Filtre de protection contre la lumière, pour écrans ou lunettes de soudage, comportant un élément filtrant commandable électriquement en ce qui concerne sa transmission optique et accouplé à un dispositif de commande de transmission qui comprend au moins un capteur photosensible placé après l'élément filtrant dans le sens d'incidence de la lumière et qui règle l'élément filtrant à différentes valeurs de transmission en fonction inverse de l'intensité de la lumière qui tombe sur le capteur, un transducteur optoélectrique étant prévu comme source de courant d'alimentation pour la commande de la transmission, caractérisé en ce que le capteur est placé en aval d'un filtre IR et éventuellement en aval d'un filtre UV.

2. Filtre de protection contre la lumière, pour écrans ou lunettes de soudage, comportant un dispositif filtrant commandable électriquement en ce qui concerne sa transmission optique, constitué de deux éléments filtrants placés l'un derrière l'autre et accouplé à un dispositif de commande de transmission qui comprend au moins un capteur photosensible placé après le dispositif filtrant dans le sens d'incidence de la lumière et qui règle le dispositif filtrant à différentes valeurs de transmission en fonction inverse de l'intensité de la lumière qui tombe sur le capteur, un transducteur optoélectrique étant prévu comme source de courant d'alimentation pour la commande de la transmission, caractérisé en ce que le capteur est placé en aval d'un filtre IR et en aval du premier élément filtrant du dispositif filtrant, mais en amont du second, et éventuellement en aval d'un filtre UV.

3. Filtre de protection contre la lumière selon l'une des revendications 1 et 2, caractérisé en ce que le capteur pour la commande de la transmission est formé par l'élément transducteur optoélectrique.

Fig.1

Fig.2

Fig. 3

Fig.4

Fig.5.